**Europäisches Patentamt**

**European Patent Office**

**Office européen des brevets**

(19)

(11) Veröffentlichungsnummer: **0 330 012 B1**

(12)

# EUROPÄISCHE PATENTSCHRIFT

(45) Veröffentlichungstag der Patentschrift :
09.10.91 Patentblatt 91/41

(51) Int. Cl.⁵ : **A61M 25/00**

(21) Anmeldenummer : **89102160.2**

(22) Anmeldetag : **08.02.89**

(54) **Medizinischer Katheter für die intravaskuläre Langzeitinfusion von Medikamenten.**

(30) Priorität : 22.02.88 DE 3805508

(43) Veröffentlichungstag der Anmeldung :
30.08.89 Patentblatt 89/35

(45) Bekanntmachung des Hinweises auf die
Patenterteilung :
09.10.91 Patentblatt 91/41

(84) Benannte Vertragsstaaten :
DE FR GB IT SE

(56) Entgegenhaltungen :
EP-A- 0 052 841
EP-A- 0 185 865
DE-A- 1 491 682
US-A- 4 464 178
US-A- 4 516 970
US-A- 4 705 501

(73) Patentinhaber : SIEMENS
AKTIENGESELLSCHAFT
Wittelsbacherplatz 2
W-8000 München 2 (DE)

(72) Erfinder : Franetzki, Manfred, Dr.
Schleifweg 7B
W-8525 Uttenreuth (DE)
Erfinder : Funke, Helmut
Gässlein 21
W-8521 Möhrendorf (DE)
Erfinder : Schweikert, Eugen
Sudetenstrasse 34
W-8526 Bubenreuth (DE)

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen medizinischen Katheter für die intravaskuläre Langzeitinfusion von Medikamenten durch implantierte oder extrakorporale Dosiergeräte nach dem Oberbegriff des Patentanspruchs 1. Solche aus mehrschichtigem Kunststoffmaterial aufgebauten medizinischen Arbeitsmittel sind aus der DE-PS 3204762 bekannt.

Bei Langzeitinfusionen von Medikamenten, beispielsweise Insulin, in den menschlichen Körper durch extern tragbare oder implantierbare Dosiergeräte ist deren Ausgang an einen Katheter angeschlossen, dessen Öffnung vorzugsweise im Peritoneum (interperitonal) oder einer großen Vene (intravenös) plaziert ist. Bei einer solchen Langzeitkatheterisierung ergeben sich Probleme, die mit der Fremdkörperreaktion des menschlischen Körpers zusammenhängen. Im wesentlichen treten dabei die folgenden drei Reaktionsmuster auf :

a) von der Einführungsstelle des Katheters in das Peritoneum bzw. das Blutgefäß oder von Berührungsstellen mit diesen aus wächst eine Bindegewebehaut in Richtung auf die Katheterspitze und umwächst diese ;

b) von der Katheterspitze aus wächst Bindegewebe in distaler Richtung ;

c) Körperzellen, Makromoleküle und Gewebeteilchen dringen in die Katheteröffnung ein und lagern sich an deren Wänden an.

Dabei hängt die Haftfähigkeit solcher Aufwachsungen in besonderm Maße von der Oberflächenrauhigkeit ab. Die Fremdkörperreaktionen werden durch Reize chemischer Art einmal vom körperfremden Material selbst, zum anderen aber auch durch die infundierte Flüssigkeit ausgelöst. Darüber hinaus tritt ein mechanischer Reiz etwa durch die Steifigkeit und die Kanten des Katheters als Auslöser auf. Über den Anteil der vorerwähnten Reizwirkungen an der Fremdkörperreaktion ist nichts bekannt. Allerdings ist die Zuwachsgeschwindigkeit häufig sehr viel kürzer als die Standzeit des implantierten Dosiergerätes, so daß es zu einem vorzeitigen Funktionsausfall mit der Notwendigkeit einer chirurgischen Katheterrevision kommt.

Aus der EP-A-0185865 ist ein implantierbarer intraperitonealer Katheter bekannt, der sich unter anderem für die Injektion von Insulin in den menschlichen Körper eignet. Dabei ist die Katheterspitze zur Vermeidung von Verstopfungen durch Aufwachsungen von Körpergewebe mit hintereinander angeordneten Austrittslöchern versehen, zwischen denen sich seitlich abstehende scheibenförmige Distanzelemente befinden. Der Nachteil dieser bekannten Anordnung besteht vor allem darin, daß die seitlich überstehenden Distanzscheiben zum einen das Einführen des Katheters erschweren und während des Einführungsvorganges die Gefäßinnenwände verletzen können, und zum anderen im stationären Zustand durch Wirbelbildungen des Blutstromes in Flußrichtung hinter den überstehenden Distanzelementen Gefäßüberwucherungen verursachen.

Aus der US-A-4705501 ist ein weiterer Katheter der vorgenannten Art bekannt, dessen distales Ende vom Druck der einströmenden Flüssigkeit bzw. vom Gegendruck der im Gefäß strömenden Flüssigkeit geöffnet und geschlossen werden kann. Zu diesem Zweck ist das distale Ende des Katheters in einem spitzen Winkel derart abgeschrägt, daß eine zwischen dem stumpfen Winkel des abgeschrägten Endes und dessen spitzem Winkel angeordnete bewegliche Zunge in Abhängigkeit von den jeweiligen Strömungsverhältnissen den Katheter öffnet oder schließt. Bei Kathetern, die mit einer Dosierpumpe verbunden sind, ist ein solcher Ventilmechanismus überflüssig. Außerdem trägt die bewegliche Zunge zu einer verstärkten Gewebereizung bei.

Aus der DE-A-1491682 ist schließlich ein Verfahren zur Herstellung von Kathetern mit verbesserter physiologischer Verträglichkeit bekannt, dessen Oberflächen mit einer Schicht aus Organo-Polysiloxanelastomer überzogen sind. Solche Polysiloxanelastomere weisen als Füllstoff Silikagel auf. Es hat sich gezeigt, daß dieses Material mit Gewebe und Blut reagiert, so daß sich Proteine, Trombozyten und — wegen der relativ rauhen Oberfläche — auch Bindegewebe anlagern.

Der Erfindung liegt die Aufgabe zugrunde, den Katheter so zu gestalten, daß sich dessen Funktionszeit deutlich verlängert, und möglichst die Standzeit des implantierten Dosiergerätes übersteigt.

Diese Aufgabe wird durch die im Patentanspruch 1 angegebene Erfindung gelöst. Durch die axiale Versetzung der Medikamenten- Austrittsöffnung gegenüber der Katheterspitze ist erreicht, daß ein Katheterverschluß durch von der Katheterspitze aus beginnendes Wachstum von Körpergewebe zumindest erheblich verzögert wird. Durch die nachträgliche Beschichtung des Katheters mit Hydrogel werden scharfe Kanten und Spitzen verrundet. Außerdem hat der Hydrogelfilm eine glatte Oberfläche. Dadurch werden Gewebeüberwucherungen stark reduziert.

Die Ausführungsform nach Patentanspruch 3 hat den Vorteil, daß auch nach einem etwaigen Zuwachsen der Austrittsöffnung keine Unterbrechung der Medikamentenzufuhr stattfindet. Die am Umfang der Katheterspitze angeordneten Längsschlitze bewirken nämlich, daß durch den Druckanstieg innerhalb des Katheters und die dadurch verursachte Tangentialkraft die in die Mantelfläche eingebrachten Schlitze aufspreizen, so daß das Medikament einen Weg nach außen findet.

Es ist zwar aus der GB-PS 1417013 bekannt, zum Einführen von Medikamenten in das zu katheterisierende Gefäß die Katheterspitze mit seitlichen Schlitzen als Medikamentenaustrittsöffnungen zu versehen. Diese dienen aber nicht dem Zweck, den Notaustritt von Medikamenten bei verstopfter Hauptausführungsöffnung zu ermöglichen. Sie haben vielmehr die Funktion von Ventilen, um einen Rückfluß von Körperflüssigkeit durch den Katheter nach außen zu verhindern.

Weitere Einzelheiten der Erfindung werden anhand von Ausführungsbeispielen im folgenden näher erläutert. Es zeigen :

FIG 1        die Spitze eines Vollkunststoff-Katheters ;
FIG 2 und 3        eine Katheterspitze mit distaler Öffnung und seitlichen Schlitzen als Notöffnungen.

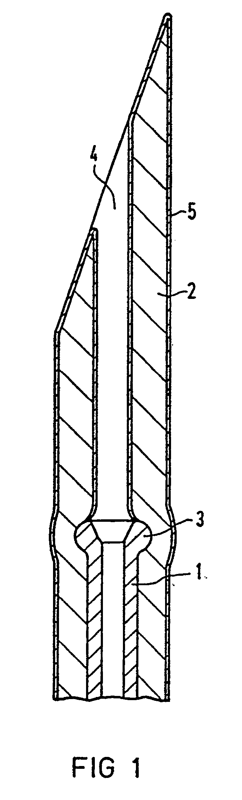

In FIG 1 ist das als Katheterspitze ausgebildete distale Ende eines nicht näher dargestellten Katheterschlauches dargestellt. Bei dem dargestellten Katheter handelt es sich um einen Verbundschlauch, bei dem über einen Kernschlauch aus Polyäthylen 1 ein Elastomerschlauch 2 aus Silikon aufgeschoben ist. Dazu ist das Ende des Kernschlauches 1 mit einer umlaufenden Wulst 3 versehen, die den aufgeschobenen Silikonschlauch 2 gegen Abstreifen sichert. Die Spitze des Silikonschlauches 2 ist unter einem spitzen Winkel (z.B. 15°) abgeschnitten. Damit ist erreicht, daß zum einen die Katheterspitze sehr weich wird, also das Körpergewebe weniger mechanisch reizt, und daß zum anderen die distale Katheteröffnung 4 einen axialen Abstand von der Spitze hat mit der Folge, daß bei eventuellem Wachstum von Körpergewebe von der Spitze her ein Katheterverschluß zumindest verzögert wird. Der Katheter ist nun zur Erreichung einer besseren biologischen Verträglichkeit einerseits und zur Herstellung einer glatten Oberfläche andererseits durch eine Schutzschicht 5 aus einem Hydrogel, beispielsweise Polyäthylenglykol, überzogen. Die Beschichtung kann durch Eintauchen des fertig konfektionierten Katheters in das Polyäthylenglykol erzeugt werden. Ein solcher Hydrogelfilm hat eine glatte Oberfläche und enthält keine Füllstoffe. Gleichzeitig wird damit erreicht, daß die beim Schneiden des Silikonschlauches entstandenen scharfen Kanten und Spitzen verrundet werden. Anstelle von Polyäthylenglykol können zur Beschichtung auch andere weiche und körperfreundliche Materialien wie füllerfreies Polysiloxan verwendet werden.

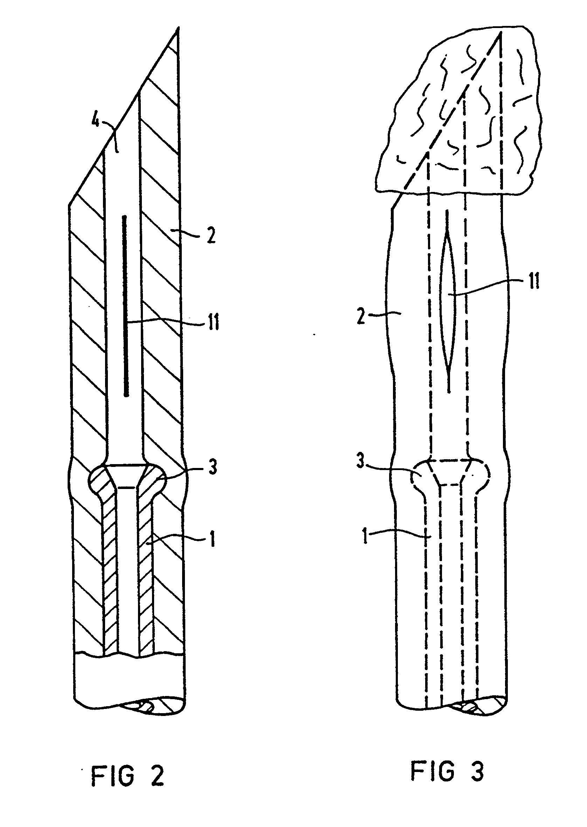

Die in FIG 2 dargestellte Katheterspitze entspricht weitgehend derjenigen nach FIG 1. Zusätzlich jedoch sind in den Silikonschlauch 2 unterhalb der distalen Katheteröffnung 4 Längsschlitze 11 eingebracht, die sich bei einem eventuellen Verschluß der distalen Öffnung 4 durch Gewebüberwucherung, wie in FIG 3 dargestellt, unter dem Druck des eingepumpten Medikamentes öffnen. Damit ist erreicht, daß die Medikamentenzufuhr auch dann nicht unterbrochen wird, wenn ein solcher Verschluß der Hauptöffnung eingetreten ist. Damit besitzt der Katheter Notbetriebseigenschaften, die seine Standfestigkeit erheblich verlängern.

## Patentansprüche

1. Medizinischer Katheter für die intravaskuläre Langzeitinfusion von Medikamenten durch implantierte oder extrakorporale Dosiergeräte mit einem als Verbundschlauch ausgebildeten Katheterschlauch, der einen äußeren, gegenüber dem darunterliegenden Elastomerschlauch weichen und nach außen hin toxikologisch einwandfreien und biologisch verträglichen Schlauch (2) aufweist, **dadurch gekennzeichnet,** daß die Katheterspitze ausschließlich von dem äußeren Elastomerschlauch (2) gebildet wird, der unter einem spitzen Winkel abgeschnitten ist, so daß die Austrittsöffnung (4) für das Medikament gegenüber dem distalen Ende des Katheters in axialer Richtung rückversetzt angeordnet ist, und daß der fertig konfektionierte Katheter mit einer äußeren Schutzschicht (5) aus einem Hydrogel überzogen ist.

2. Medizinischer Katheter nach Anspruch 1, **dadurch gekennzeichnet,** daß das Hydrogel aus Polyäthylenglykol oder füllerfreiem Polysiloxan besteht.

3. Medizinischer Katheter nach Anspruch 1 oder 2, **dadurch gekennzeichnet,** daß der äußere Elastomerschlauch (2) im Bereich der Katheterspitze zusätzliche längsschlitzförmige seitliche Öffnungen (11) als Notaustrittsöffnungen für den Medikamentenaustritt bei verstopfter Hauptaustrittsöffnung (4) besitzt.

## Claims

1. Medical catheter for the intravascular long-term infusion of medicaments by implanted or extracorporeal dosage devices, with a catheter tube which is designed as a composite tube and has an external elastomer tube (2) which is soft compared to the tube lying below and is externally toxicologically safe and biologically compatible, characterised in that the catheter tip is formed exclusively from the external elastomer tuba (2) which is cut off at an acute angle so that the exit opening (4) for the medicament is arranged offset in the axial direction relative to the distal end of the catheter, and in that the finished catheter is coated with an external protective layer (5) composed of a hydrogel.

2. Medical catheter according to claim 1, characterised in that the hydrogel consists of polyethylene glycol or of polysiloxane free of filler.

3. Medical catheter according to claim 1 or 2, characterised in that the external elastomer tube (2) has, in the area of the catheter tip, additional longitudinal slot-shaped lateral openings (11) as emergency exit openings for the medicament discharge in the event of the main exit opening (4) becoming blocked.

## Revendications

1. Cathéter médical pour l'administration intravasculaire de longue durée de médicaments au moyen d'appareils de dosage implantés ou extracorporels, comportant un tuyau de cathéter agencé sous la forme d'un tuyau composite et comportant un tuyau élastomère extérieur (2), qui est mou par rapport au tuyau sous-jacent et est parfait du point de vue toxicologique vers l'extérieur et compatible du point de vue biologique, caractérisé par le fait que la pointe du cathéter est formée exclusivement par le tuyau extérieur élastomère (2), qui est sectionné sous un angle aigu de sorte que l'ouverture de sortie (4) pour le médicament est disposée axialement en retrait, par rapport à l'extrémité distale du cathéter, et le cathéter terminé est recouvert d'une couche extérieure protectrice (5) formée d'un hydrogel.

2. Cathéter médical suivant la revendication 1, caractérisé par le fait que l'hydrogel est formé par du polyéthylèneglycol ou du polysiloxane ne contenant aucune charge.

3. Cathéter médical suivant la revendication 1 ou 2, caractérisé par le fait que le tuyau extérieur élastomère (2) possède, au niveau de la pointe du cathéter, des ouvertures latérales supplémentaires (11) en forme de fentes allongées constituant des ouvertures de sortie d'urgence pour le passage du médicament dans le cas où l'ouverture principale de sortie (4) serait obstruée.

FIG 1

FIG 2

FIG 3